(19) Europäisches Patentamt
European Patent Office
Office européen des brevets

(11) Numéro de publication : **0 565 450 A1**

(12) **DEMANDE DE BREVET EUROPEEN**

(21) Numéro de dépôt : **93400924.2**

(51) Int. Cl.$^5$ : **A61K 37/02,** C07K 5/08

(22) Date de dépôt : **08.04.93**

(30) Priorité : **10.04.92 FR 9204429**

(43) Date de publication de la demande :
**13.10.93 Bulletin 93/41**

(84) Etats contractants désignés :
**CH DE DK FR GB IT LI**

(71) Demandeur : **COMMISSARIAT A L'ENERGIE ATOMIQUE**
**31-33, rue de la Fédération**
**F-75015 Paris (FR)**

(72) Inventeur : **Dive, Vincent**
**17, Avenue Georges Clémenceau**
**F-94300 Vincennes (FR)**
Inventeur : **Toma, Flavio**
**14, rue de Normandie**
**F-92140 Clamart (FR)**
Inventeur : **Yiotakis, Athanasios**
**7, rue Paradisou**
**15343 AG. Paraskevi, Athenes (GR)**

(74) Mandataire : **Des Termes, Monique et al**
**Société Brevatome 25, rue de Ponthieu**
**F-75008 Paris (FR)**

(54) **Utilisation de dérivés de peptides pour la fabrication de médicaments inhibiteurs des endopeptidases 24.15 et 24.16.**

(57) L'invention concerne l'utilisation de dérivés de peptides pour la fabrication de médicaments inhibiteurs des endopeptidases 24.15 et 24.16.

Ces dérivés de peptides répondent à la formule :

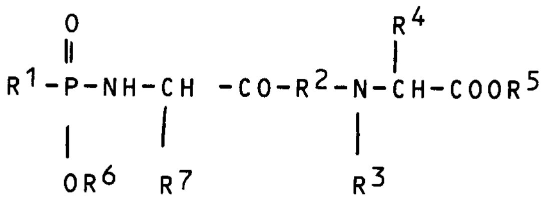

(I)

dans laquelle $R^1$ est un groupe aralkyle éventuellement substitué, $R^2$ est Pro, Hyp, thiazolidine ou déshydroproline, $R^3$ est H ou un radical alkyle, $R^4$ est un groupe alkyle ou la chaîne latérale d'un aminoacide, $R^5$ et $R^6$ peuvent être H, $NH_4^+$ ou des métaux, et $R^7$ est H ou $CH_3$.

L'acide N-(phényléthylphosphonyl)-Gly-Pro-aminohexanoïque et l'acide N-(phényléthyl-phosphonyl)-Ala-Pro-aminohexanoïque sont des exemples de dérivés efficaces.

EP 0 565 450 A1

La présente invention a pour objet l'utilisation de dérivés de peptides du type phosphonamide pour la fabrication de médicaments inhibiteurs des endopeptidases 24.15 et/ou 24.16.

Les endopeptidases sont des enzymes appartenant à la famille des métallopeptidases contenant du zinc qui présentent la propriété d'inactiver certains neuromodulateurs agissant au niveau du cerveau tels que la neurotensine, et de détruire ainsi leurs effets pharmacologiques.

Aussi, des inhibiteurs de ces endopeptidases seraient d'un grand intérêt pour pouvoir potentialiser les effets pharmacologiques associés à la neurotensine.

On sait que certains dipeptides tels que Pro-Ile sont capables d'inhiber l'endopeptidase 24.16, comme il est décrit par Dauch et al dans Eur. J. Biochem., vol 202, p. 269-276, 1991b. Cependant, cet inhibiteur de l'endopeptidase 24.16 a une constante d'inhibition Ki de 90μM, qui est beaucoup trop élevée, compte tenu de la solubilité de ce dipeptide, pour permettre son utilisation "in vivo". Par ailleurs, il n'a aucun effet inhibiteur sur l'endopeptidase 24.15 à une concentration aussi élevée que 5mM.

Pour l'endopeptidase 24.15, on connaît toutefois un composé inhibiteur de cette enzyme qui est un peptide de formule :

CPP-Ala-Ala-Tyr-pAB

avec CPP représentant le radical carboxy-3-phénylpropyl et pAB représentant le p-aminobenzoate, présentant une constante d'inhibition de 16nM, comme il est décrit par Orlowski et al, Biochemistry, vol 27, p. 597-602, 1988. Cet inhibiteur est donc beaucoup plus efficace que l'inhibiteur Pro-Ile de l'endopeptidase 24.16 mais il ne concerne pas l'endopeptidase 24.16. Il a toutefois un effet inhibiteur sur l'endopeptidase 24.16 qui correspond à une constante d'inhibition de 1μM beaucoup trop élevée, comme il est décrit par Dauch et al dans Biochem. J., 280, 1991, p. 421-426.

Aussi, des recherches ont été entreprises pour trouver d'autres inhibiteurs plus efficaces vis-à-vis des endopeptidases 24.16 et 24.15 qui hydrolysent toutes deux la neurotensine.

A la suite de ces recherches, on a trouvé que certains dérivés de peptides décrits dans FR-A-2.654 430, connus pour avoir un effet inhibiteur sur des collagénases bactériennes telles que la collagénase de Clostridium Histolyticum présentaient un effet inhibiteur encore plus marqué et très spécifique sur les endopeptidases 24.16 et 24.15. Cet effet est tout-à-fait inattendu car les endopeptidases possèdent une spécificité différente de celle des collagénases bactériennes et elles n'ont aucune similitude reconnue avec les collagénases bactériennes.

Aussi, la présente invention a pour objet l'utilisation d'un dérivé de peptide de formule :

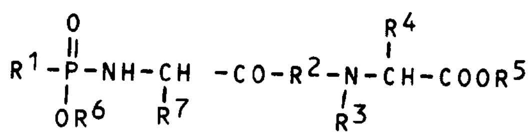 (I)

dans laquelle

- $R^1$ représente un groupe aralkyle dont le groupe alkyle a 1 à 3 atomes de carbone, le groupe aralkyle étant non substitué ou substitué sur sa partie aryle par au moins un substituant choisi parmi les groupes halo, trifluorométhyl, alcoxy en $C_1$ à $C_4$, alkyle en $C_1$ à $C_4$, alcanoyloxy en $C_1$ à $C_4$, carbonyloxyalkyle en $C_2$ à $C_5$, nitro, carboxy ou cyano ;
- $R^2$ est un radical bivalent dérivé d'un aminoacide choisi parmi la proline, l'hydroxyproline, la thiazolidine et la déshydroproline, de formule :

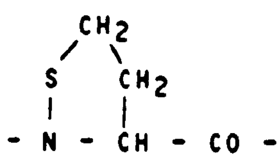

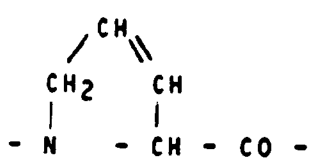

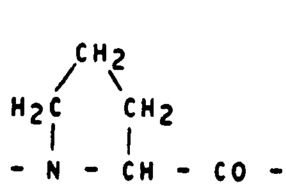

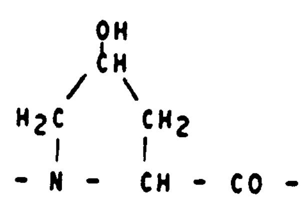

relié par sa partie NH à CO ;

- $R^3$ est un atome d'hydrogène ou un groupe alkyle en $C_1$ à $C_4$,
- $R^4$ est un groupe alkyle en $C_1$ à $C_5$ ou la chaîne latérale d'un α-aminoacide ;
- $R^5$ représente un atome d'hydrogène, $NH_4^+$, un atome de métal pharmaceutiquement acceptable, un groupe alkyle en $C_1$ à $C_5$ ou un groupe benzyle ;
- $R^6$ représente un atome d'hydrogène, $NH_4^+$, ou un atome de métal pharmaceutiquement acceptable, et
- $R^7$ représente H ou $CH_3$,

pour la fabrication d'un médicament inhibiteur des endopeptidases 24-15 et/ou 24-16.

Le médicament inhibiteur des endopeptidases 24.15 et/ou 24.16 est un médicament potentialisant les effets pharmacologiques associés à la neurotensine.

Ce médicament peut donc être un analgésique, un agent antihypertenseur ou un agent hypothermiant.

Dans les dérivés de peptides décrits ci-dessus, les termes "aryle", "aralkyle", et "α amino-acide" ont les significations suivantes.

Le terme "aryle" désigne des systèmes à noyau aromatique carbocyclique et hétérocyclique contenant par exemple un ou plusieurs hétéroatomes tels que O, S et N. Ces groupes aryle ont généralement de 3 à 10 atomes de carbone.

A titre d'exemples de groupes aryle, on peut citer les groupes phényle, naphtyle, furyle, thiényle, pyrolyle, imidazolyle, pyridyle, pyrimidinyle, indolyle, quinolyle, oxazolyle et isooxazolyle.

Le terme "aralkyle" désigne des groupements formés par l'association d'un groupe aryle et d'un groupe alkyle. Les groupes aryle peuvent être du type de ceux décrits ci-dessus. Les groupes alkyle peuvent être linéaires ou ramifiés et ont de 1 à 3 atomes de carbone.

Le groupe aralkyle peut être substitué sur sa partie aryle par au moins un substituant choisi parmi les groupes halo, par exemple le fluor, le chlore, le brome ou l'iode, trifluorométhyle, alcoxy en $C_1$ à $C_4$, alkyle en $C_1$ à $C_4$, alkanoyloxy en $C_1$ en $C_4$, carbonyloxyalkyle avec un groupe alkyle en $C_1$ à $C_4$, nitro, carboxy, et cyano.

De même, lorsque $R^1$ représente un groupe aryle, il peut être également substitué par les groupements décrits ci-dessus.

Le terme "α-aminoacide" utilisé ici se rapporte aux vingt α-aminoacides communément trouvés dans les protéines qui sont également connus sous le nom d'aminoacides standard et à leurs analogues. Les chaînes latérales de ces aminoacides comprennent des groupes alkyle linéaires et ramifiés, hydroxyalkyle, carboxyalkyle, aralkyle, aminoalkyle, carboxamide alkyle, mercapto alkyle, phénylalkyle, hydroxyphénylalkyle, guanidinoalkyle, imidazoylalkyle, indolylalkyle, et pyrrolidinyle.

A titre d'exemple d'acides aminés utilisables, on peut citer l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la tyrosine, la valine, la nitrophényl alanine, l'homo arginine, la thiazolidine et la déshydroproline.

Dans les dérivés de peptides, les métaux utilisables pour $R^5$ et $R^6$ sont des métaux pharmaceutiquement acceptables tels que les métaux alcalins, en particulier le sodium et le lithium.

Les dérivés de peptides utilisés dans l'invention peuvent être préparés par des procédés classiques, en particulier par le procédé décrit dans FR-A- 2 654 430.

Selon l'invention, pour la fabrication du médicament inhibiteur des endopeptidases 24.15 et/ou 24.16, on inclut une quantité pharmaceutiquement efficace du dérivé de peptide de formule (I) donnée ci-dessus, à l'état pur ou sous la forme de sel pharmaceutiquement acceptable, dans un véhicule ou un support physiologiquement acceptable approprié. Ce support peut être une solution ou une suspension pour injections.

On peut aussi utiliser des supports et des excipients appropriés pour obtenir un médicament destiné à l'administration orale sous la forme de comprimés ou de capsules, incorporant de plus, si nécessaire, des additifs classiques tels que le carbonate de magnésium, le stéarate de magnésium, le talc, le sucre, le lactose, la pectine, la dextrine, l'amidon, la gélatine, le tragacanthe, la méthylcellulose, la carboxyméthylcellulose de sodium, le beurre de cacao etc. Pour cette fabrication, on peut aussi ajouter des diluants, des agents de saveurs, des solubilisants, des lubrifiants, des agents de mise en suspension, des liants et des agents de désintégration.

Généralement, le médicament est administré à des doses se situant dans la gamme allant de 0,1mg/kg/jour à environ 5mg/kg/jour du dérivé de peptide de formule (I).

Il peut être utilisé en tant qu'agent analgésique ou hypothermiant, ou pour le traitement de certaines hypertensions artérielles.

D'autres caractéristiques et avantages de l'invention apparaîtront mieux à la lecture des exemples suivants donnés bien entendu à titre illustratif et non limitatif, en référence au dessin annexé, sur lequel

- la figure 1 est une courbe illustrant l'effet inhibiteur (en %) du dérivé de peptide sur l'endopeptidase 24.16 en fonction de la concentration en inhibiteur,
- les figures 2 à 7 sont les chromatogrammes obtenus lors d'essais d'inhibition de l'endopeptidase 24.16,

- la figure 8 illustre des courbes représentant l'effet inhibiteur (en %) d'un dérivé de l'invention sur l'endopeptidase 24.16 (courbe 1) et sur l'endopeptidase 24.15 (courbe 2) en fonction de la concentration en inhibiteur, et
- la figure 9 est une courbe illustrant l'effet inhibiteur (en %) du dérivé de l'invention sur l'endopeptidase 24.15 en fonction de la concentration en inhibiteur.

**Exemple 1** :Préparation du dérivé de peptide de formule :

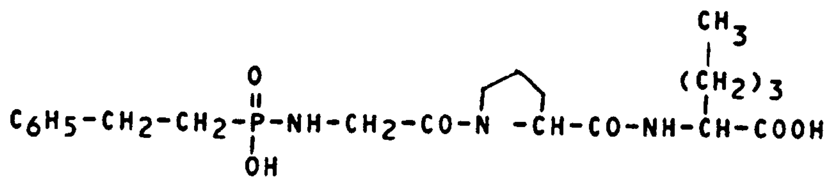

Ce dérivé que l'on désignera ci-après sous le terme de "phosphodiépryl 03", est un dérivé répondant à la formule (I) dans laquelle $R^1$ est le groupe phényléthyle, $R^2$ est dérivé de la proline, $R^3$ est un atome d'hydrogène, $R^4$ est le groupe n-butyle et $R^5$ est un atome d'hydrogène.

a) Préparation du phosphonate du dibenzylphényléthyle :

$$C_6H_5\text{-}CH_2\text{-}CH_2\text{-}P\text{-}(OCH_2C_6H_5)_2$$

On mélange délicatement dans du diméthylformamide 1mmol d'hydrure de sodium, sous atmosphère d'azote à -15°C, et on y ajoute peu à peu 1mmol de phosphite de dibenzyle dissoute dans du diméthylformamide. On laisse la réaction se poursuivre jusqu'à l'arrêt complet du dégagement d'hydrogène.

On ajoute alors sous agitation 1mmol de bromure de phényléthyle dans du diméthylformamide, toujours sous atmosphère d'azote, et on réalise cette addition suffisamment lentement pour que la température varie de -10°C à 0°C. Quand toute la solution de bromure de phényléthyle est ajoutée, on laisse le mélange réactionnel revenir à 25°C. On enlève l'azote et on maintient sous agitation pendant 3h.

On évapore alors de ce mélange le diméthylformamide (DMF) sous vide poussé, à une température de 35 à 40°C, puis on reprend dans de l'éther, on lave ensuite deux fois à l'eau la phase organique, puis on la sèche sur sulfate de sodium. Après évaporation du solvant, on obtient le dibenzylphényléthyl phosphonate voulu avec un rendement de 80%.

On purifie ensuite ce produit par chromatographie flash sur silice en utilisant comme éluant un mélange d'éther et d'acétate d'éthyle dans un rapport 2/3 en volume. On obtient ainsi le phosphonate purifié avec un rendement de 40%.

b) Préparation du chlorure de monobenzylphényléthyl phosphonate.

On ajoute 1mmol de pentachlorure de phosphore dans du benzène à une solution dans du benzène de 1mmol du dibenzylphényléthyl phosphonate obtenu précédemment. On chauffe le mélange à 70°C sous reflux et sous atmosphère anhydre, pendant 3 à 4h, jusqu'à l'obtention complète du chlorure. On évapore ensuite le benzène de ce mélange et on le reprend dans du dichlorométhane sec. On utilise immédiatement cette solution pour réaliser le couplage avec le peptide.

c) Couplage avec le peptide.

A la solution de 1mmol de chlorure de monobenzylphényléthyl phosphonate dans du dichlorométhane obtenue précédemment, on ajoute à 0°C, 0,9mmol du peptide HCl, Gly-Pro-Nle-$OCH_2$-$C_6H_5$ dans du dichlorométhane et deux équivalents de triéthylamine. On laisse réagir sous agitation pendant une demi-heure toujours à 0°C, puis pendant une demi-heure supplémentaire à 25°C. On évapore alors le dichlorométhane et on reprend l'huile dans de l'acétate d'éthyle, puis on lave à l'eau avec de l'acide chlorhydrique 0,1N, du $NaHCO_3$ à 5%, puis avec de l'eau jusqu'à neutralité. On sèche la phase organique puis on l'évapore. On obtient ainsi le dérivé de peptide protégé avec un rendement de 90%. On purifie ce dérivé par chromatographie flash en utilisant comme solvant d'élution le mélange dichlorométhaneméthanol (9/1 en volume).

d) Déprotection.

On réalise la déprotection du dérivé par hydrogénation catalytique en utilisant le palladium comme catalyseur. On obtient ainsi le phosphodiépryl 03.

**Exemples 2 à 7** : Préparation de dérivés de peptides.

Dans ces exemples, on suit le même mode opératoire que dans l'exemple 1 pour préparer les dérivés de peptides du tableau 1 annexé, en préparant dans l'étape a) le phosphonate correspondant par le même procédé que celui décrit pour $C_6H_5-(CH_2)_2-P-(OCH_2C_6H_5)_2$ et en réalisant dans l'étape c) un couplage avec le peptide approprié.

**Exemple 8** : Utilisation du phosphodiépryl 03 comme inhibiteur de l'endopeptidase 24.16.

On incube pendant 1h à 37°C, 5nmol (50µm/l) de Mcc-Pro-Leu-Gly-Pro-DLys-Dnp, qui est un substrat connu de l'endopeptidase 24.16, Mcc représentant le radical 7-méthoxycoumarine-3-carboxyle et Dnp représentant le radical 2,4-dinitrophényle, avec 10µl d'endopeptidase 24.16 purifié dans un volume final de 100µl du tampon Tris-HCl, 50mM, pH 7,5, en l'absence d'inhibiteur (témoin) ou en présence de phosphodiépryl 03 à des concentrations allant de $10^{-11}$ à $10^{-7}$mol/l. Après 1h d'incubation, on analyse les solutions par fluorimétrie comme il est décrit par Dauch et al, Biochem. J. 280, p. 421-426, 1991a.

Les résultats obtenus sont donnés sur la figure 1 qui représente la fluorescence (en % de la fluorescence du témoin en fonction de la concentration (en mol/l) en phosphodiépryl 03 (exprimée en cordonnée logarithmique).

Au vu de cette figure, on constate que l'endopeptidase 24.16 est inhibée par le phosphodiépryl 03 et que le taux d'inhibition croît avec la concentration en inhibiteur.

La concentration d'inhibiteur qui inhibe 50% de l'endopeptidase, IC 50, est de 1nM et la constante d'inhibition Ki est de 0,3nM.

Ainsi, les propriétés inhibitrices du phosphodiépryl 03 sont très supérieures à celles de l'inhibiteur connu Pro-Ile (Ki=90µM).

**Exemple 9** : Inhibition de l'endopeptidase 24.16.

Dans cet exemple, on étudie comme dans l'exemple 8, l'effet inhibiteur du phosphodiépryl 03 sur l'endopeptidase 24.16 mais on utilise dans ce cas la neurotensine comme substrat d'enzyme.

Dans ce but, on incube 2nmol (20µM) de neurotensine à 37°C avec 10µl de l'endopeptidase 24.16 dans un volume final de 100µl de tampon Tris-HCl 50mM, pH 7,5 en l'absence d'inhibiteur (témoin) ou en présence de concentrations en phosphodiépryl 03 allant de $10^{-10}$ à $10^{-6}$mol/l. Après incubation, on interrompt celle-ci par acidification et on analyse la solution par chromatographie liquide à haute performance (HPLC) en utilisant un système d'élution constitué par un gradient linéaire de 42min d'acide trifluoroacétique à 0,1% et de triéthylamine à 0,05% dans l'acétonitrile, allant de 9/1 à 3/1 comme il a été décrit par Checler et al dans Biochimie 70, p. 75-82, 1988.

Les résultats obtenus sont donnés sur les figures 2 à 7 qui représentent les chromatogrammes obtenus par mesure de la densité optique à 230nm en fonction du temps (min).

Sur la figure 2, on a représenté le chromatogramme obtenu en l'absence d'inhibiteur. Sur cette figure, on voit 3 pics qui correspondent respectivement à la neurotensine 1-10 (NT 1-10), à la neurotensine 11-13 (NT 11-13) et à la neurotensine (NT). La neurotensine 1-10 et la neurotensine 11-13 sont les produits de dégradation de la neurotensine de départ.

Sur les figures 3 à 7, on remarque que les pics correspondant à la neurotensine dégradée (neurotensine 1-10 et neurotensine 11-13) diminuent avec la concentration en inhibiteur.

Sur la figure 8, (courbe 1), on a représenté le taux d'inhibition (en % par rapport au témoin) en fonction de la concentration C (en mol/l) en inhibiteur (en coordonnée logarithmique).

A partir de cette courbe, on obtient les valeurs suivantes d'inhibition :

- IC 50 = 10nM
- $K_i$ = 0,9nM.

Si l'on compare cette valeur au $K_i$ obtenu avec l'inhibiteur connu Pro-Ile (90µM), on constate que l'inhibiteur de l'invention est 100 000 fois plus efficace.

**Exemple 10** : Inhibition de l'endopeptidase 24.15.

Dans cet exemple, on étudie l'inhibition de l'endopeptidase 24.15 par le phosphodiépryl 03 en utilisant comme substrat de cette enzyme Bz-Gly-Ala-Ala-Phe-pAB avec Bz représentant le radical benzoyle et pAB le paraaminobenzoate.

Dans ce but, on incube 5nM de ce substrat à 37°C avec 10μl des fractions post-hydroxylapatite de l'endopeptidase 24.15 en l'absence (témoin) ou en présence de concentrations croissantes de phosphodiépryl 03. Après incubation, on acidifie et on analyse par chromatographie HPLC sur phase inversée en utilisant un système d'élution à gradient linéaire de 63min d'acide trifluoroacétique à 0,1% et de triéthylamine à 0,05% dans l'acétonitrile allant de 9/1 (v/v) à 5,5 / 4,5 (v/v) comme il a été décrit par Checler et al dans Biochimie 70, p. 75-82, 1988.

Les résultats obtenus sont donnés sur la figure 9.

Sur cette figure qui représente le taux d'inhibition (en % par rapport au témoin), en fonction de la concentration C (en mol/l) en inhibiteur en coordonnée logarithmique, on remarque que l'inhibition augmente régulièrement avec la concentration en inhibiteur. Les valeurs d'inhibition sont les suivantes :

- IC 50 = 5nm
- Ki = 5nM.

**Exemple 11** : Inhibition de l'endopeptidase 24.15.

Dans cet exemple, on utilise le même mode opératoire que dans l'exemple précédent, mais en utilisant la neurotensine comme substrat de l'endopeptidase 24.15.

Dans ce but, on incube 2nmol de neurotensine à 37°C avec 10μl de fractions post hydroxylapatite d'endopeptidase 24.15 en l'absence (témoin) ou en présence de concentrations croissantes de phosphodiépryl 03. Après 42min d'incubation, on acidifie et on analyse la phase liquide par chromatographie HPLC en phase inverse en utilisant des gradients linéaires du système acide trifluoroacétique , triéthylamine et acétonitrile comme dans l'exemple 3.

La courbe 2 de la figure 8 illustre les résultats obtenus.

A partir de cette courbe, on peut déterminer $IC_{50}$ et la constante d'inhibition Ki. On trouve ainsi que

- IC 50 = 100nM
- Ki = 7,5nM.

Ainsi, le phosphodiépryl 03 est également un inhibiteur très efficace de l'endopeptidase 24.15.

Dans le tableau 2 joint, on a regroupé les valeurs de Ki et de IC 50 obtenues dans les exemples 8 à 11.

**Exemple 12** : Effets du phosphodiépryl sur la carboxypeptidase A.

Dans cet exemple, on incube pendant 15min à 37°C un substrat de carboxypeptidase A constitué par 5nmol de Hippuryl-Phe avec 0,2μg de carboxypeptidase A traitée par D P dans un volume final de 100μl de Tris-HCl 50mM, pH 7,5, en l'absence ou en présence de 1μmol/l de phosphodiépryl 03. Après 15min d'incubation, on analyse la solution par HPLC.

Les résultats obtenus montrent que la présence de 1μmol/l de phosphodiépryl 03 ne donne lieu à aucune inhibition de la carboxypeptidase A.

**Exemple 13** : Effets du phosphodiépryl 03 sur la leucine aminopeptidase.

Dans cet exemple, on met à incuber 5nM d'un substrat de la leucine aminopeptidase constituée par Leu-7AMC pendant 15min, à 37°C, avec 0,02μg de leucine aminopeptidase dans un volume final de 100μl de Tris-HCl 50mM à pH 7,5, en l'absence ou en présence de 1μmol/l de phosphodiépryl 03.

Après les 15min d'incubation, on analyse le milieu réactionnel par HPLC.

Les résultats obtenus montrent qu'il n'y a eu aucune inhibition par le phosphodiépryl 03.

**Exemple 14**: Effets du phosphodiépryl 03 sur l'enzyme de conversion de l'angiotensine.

On met à incuber pendant 15min à 37°C, 5nmol d'Hippuryl-His-Leu avec 0,6μg d'enzyme de conversion de l'angiotensine dans un volume final de 100μl de Tris-HCl 50mM, pH 7,5 contenant 0,3mol/l de NaCl en l'absence ou en présence de 1μmol/l de phosphodiépryl 03.

On analyse la solution par HPLC et on constate qu'il n'y a eu aucune inhibition de l'enzyme de conversion de l'angiotensine par le phosphodiépryl 03.

**Exemple 15** : Effet du phosphodiépryl 03 sur l'endopeptidase 24.11.

On suit l'hydrolyse de 2nM de neurotensine par 9µg de l'endopeptidase 24.11 dans les conditions décrites dans l'exemple 3.

Les résultats obtenus montrent qu'il n'y a pas eu d'inhibition de l'endopeptidase 24.11 par le phosphodiépryl 03.

Ainsi, le phosphodiépryl 03 de l'invention est très intéressant car il présente un effet puissant d'inhibition vis-à-vis des endoprotéases 24.16 et 24.15 alors qu'il est sans effet sur d'autres protéases telles que la carboxypeptidase A, la leucine aminopeptidase, l'enzyme de conversion de l'angiotensine et l'endopeptidase 24.11.

Ce dérive de peptide est donc spécifique et sélectif des endopeptidases 24.15 et 24.16.

**Exemple 16** : Inhibition de l'endopeptidase 24.16.

Dans cet exemple, on étudie comme dans l'exemple 9, l'effet inhibiteur des composés des exemples 1 à 7 sur l'endopeptidase 24.16 en utilisant la neurotensine comme substrat et en opérant de la même façon sauf que l'on utilise un tampon Tris-HCl 25mM, pH 7, et une température de 25°C.

Les résultats obtenus sont donnés dans le tableau 3. Dans ce tableau, on a également indiqué les résultats obtenus avec un dérivé de peptide comportant un groupe méthyle en $R^1$ au lieu d'un groupe aralkyle.

**Exemple 17** : Inhibition de l'endopeptidase 24.15.

Dans cet exemple, on étudie comme dans l'exemple 11, l'effet inhibiteur des composés des exemples 1 à 7 sur l'endopeptidase 24.15 en utilisant la neurotensine comme substrat et en opérant dans les mêmes conditions sauf que l'on utilise un tampon Tris-HCl 25mM, pH 7 et une température de 25°C.

Les résultats obtenus sont donnés dans le tableau 3. Dans ce tableau, on a indiqué également les résultats obtenus avec le dérivé de peptide comportant un groupe méthyle en $R^1$.

Les résultats du tableau 3 montrent clairement l'efficacité des dérivés de peptide de l'invention, notamment l'effet des groupes $R^1$ et $R^2$.

En effet, lorsqu'on utilise pour $R^1$ un groupe méthyle, les résultats sont nettement inférieurs et il en est de même lorsque l'on remplace le groupe Pro par Ala.

**TABLEAU 1**

| Ex | Composé | $R^1$ | $R^2$ | $R^3$ | $R^4$ | $R^5$ | $R^6$ | $R^7$ |
|---|---|---|---|---|---|---|---|---|
| 2 | 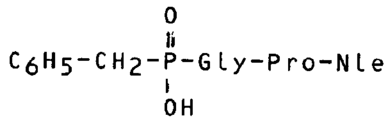 | $C_6H_5-CH_2$ | 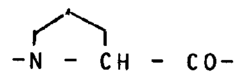 | H | $-(CH_2)_3-CH_3$ | H | H | H |
| 3 | 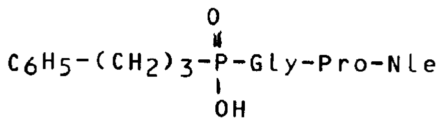 | $C_6H_5-(CH_2)_3-$ | id. | id. | id. | id. | id. | id. |
| 4 | 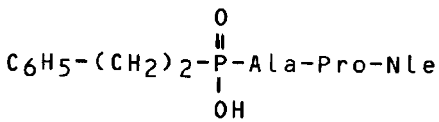 | $C_6H_5-(CH_2)_2-$ | id. | id. | id. | id. | id. | $CH_3$ |
| 5 | 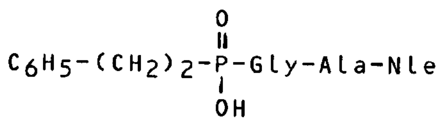 | id. | 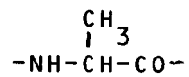 | id. | id. | id. | id. | H |
| 7 | 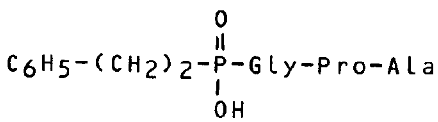 | id. | 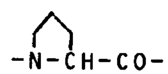 | id. | $CH_3$ | id. | id. | id. |
| 7 | 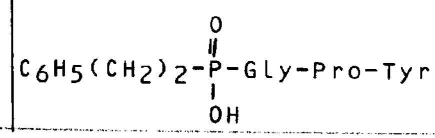 | id. | id. | id. | 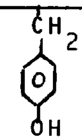 | id. | id. | id. |

**TABLEAU 2**

| Ex. | Enzyme | Substrat | $IC_{50}$ (nM) | $K_i$ (nM) |
|---|---|---|---|---|
| 8 | endopeptidase 24.16 | Mcc-Pro-Leu-Gly-Pro-DLys-Dnp | 1 | 0,3 |
| 9 | " | neurotensine | 10 | 0,9 |
| 10 | endopeptidase 24.15 | Bz-Gly-Ala-Ala-Phe-pAB | 5 | 5 |
| 11 | " | neurotensine | 100 | 7,5 |

## TABLEAU 3

| Composé | Endopeptidases | |
|---|---|---|
| | 24.16 Ki(nm) | 24.15 Ki(nm) |
| O<br>‖<br>$C_6H_5-(CH_2)_2-P-Gly-Pro-Nle$<br>\|<br>OH | 0,44 | 1,6 |
| O<br>‖<br>$C_6H_5-CH_2-P-Gly-Pro-Nle$<br>\|<br>OH | 21 | 30 |
| O<br>‖<br>$C_6H_5-(CH_2)_3-P-Gly-Pro-Nle$<br>\|<br>OH | 14 | 15 |
| O<br>‖<br>$C_6H_5-(CH_2)_2-P-Ala-Pro-Nle$<br>\|<br>OH | 0,36 | 0,48 |
| O<br>‖<br>$C_6H_5-(CH_2)-P-Gly-Ala-Nle$<br>\|<br>OH | 27 | 50 |
| O<br>‖<br>$C_6H_5-(CH_2)_2-P-Gly-Pro-Ala$<br>\|<br>OH | 3 | 6 |
| O<br>‖<br>$C_6H_5(CH_2)_2-P-Gly-Pro-Tyr$<br>\|<br>OH | 5 | 7 |
| O<br>‖<br>$CH3-P-Gly-Pro-Nle$<br>\|<br>OH | 105 | 223 |

**Revendications**

1. Utilisation d'un dérivé de peptide de formule :

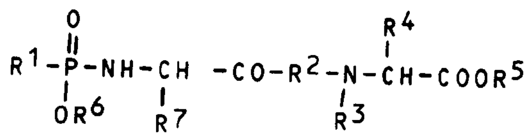 (I)

dans laquelle

- $R^1$ représente un groupe aralkyle dont le groupe alkyle a 1 à 3 atomes de carbone, le groupe aralkyle étant non substitué ou substitué sur sa partie aryle par au moins un substituant choisi parmi les groupes halo, trifluorométhyl, alcoxy en $C_1$ à $C_4$, alkyle en $C_1$ à $C_4$, alcanoyloxy en $C_1$ à $C_4$, carbonyloxyalkyle en $C_2$ à $C_5$, nitro, carboxy ou cyano ;
- $R^2$ est un radical bivalent dérivé d'un aminoacide choisi parmi la proline, l'hydroxyproline, la thiazolidine et la déshydroproline, de formule :

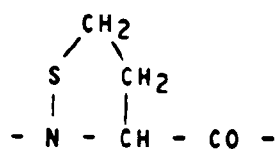
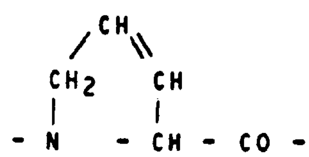
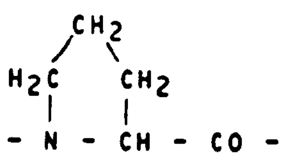
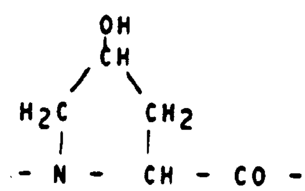

relié par sa partie NH à CO ;
- $R^3$ est un atome d'hydrogène ou un groupe alkyle en $C_1$ à $C_4$,
- $R^4$ est un groupe alkyle en $C_1$ à $C_5$ ou la chaîne latérale d'un $\alpha$-aminoacide ;
- $R^5$ représente un atome d'hydrogène, $NH_4^+$, un atome de métal pharmaceutiquement acceptable, un groupe alkyle en $C_1$ à $C_5$ ou un groupe benzyle ;
- $R^6$ représente un atome d'hydrogène, $NH_4^+$, ou un atome de métal pharmaceutiquement acceptable ; et
- $R^7$ représente H ou $CH_3$,

pour la fabrication d'un médicament inhibiteur des endopeptidases 24-15 et/ou 24-16.

2. Utilisation selon la revendication 1, caractérisé en ce que le médicament est un analgésique, un agent antihypertenseur inhibiteur des endopeptidases 24-15 et/ou 24-16, ou un agent hypothermiant.

3. Utilisation selon l'une quelconque des revendications 1 et 2, caractérisé en ce que $R^2$ est le radical dérivé de la proline de formule :

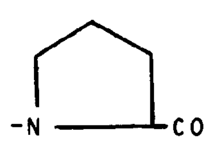

4. Utilisation selon l'une quelconque des revendications 1 à 3, caractérisée en ce que $R^1$ est le groupe benzyle, phényléthyle ou phénylpropyle.

5. Utilisation selon l'une quelconque des revendications 1 à 4, caractérisée en ce que $R^3$ est un atome d'hydrogène et $R^4$ est le groupe n-butyle, le groupe méthyle ou le groupe p-hydroxyphénylméthyle.

6. Utilisation selon l'une quelconque des revendications 1 à 5, caractérisée en ce que $R^5$ et $R^6$ sont H.

7. Utilisation selon l'une quelconque des revendications 1 et 2, caractérisée en ce que le dérivé de peptide répond à la formule :

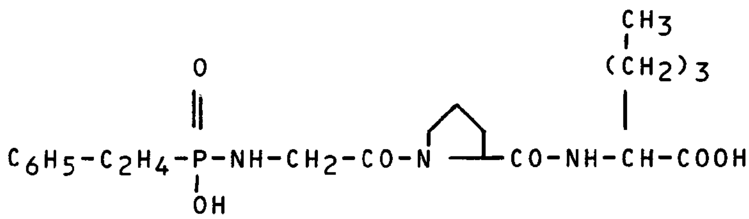

8. Utilisation selon l'une quelconque des revendications 1 et 2, caractérisée en ce que le dérivé de peptide répond à la formule :

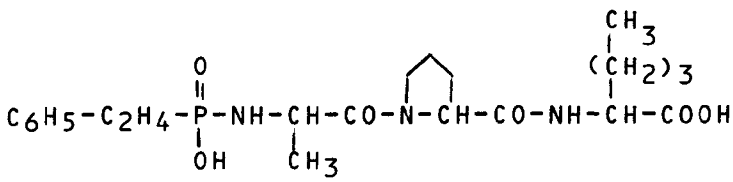

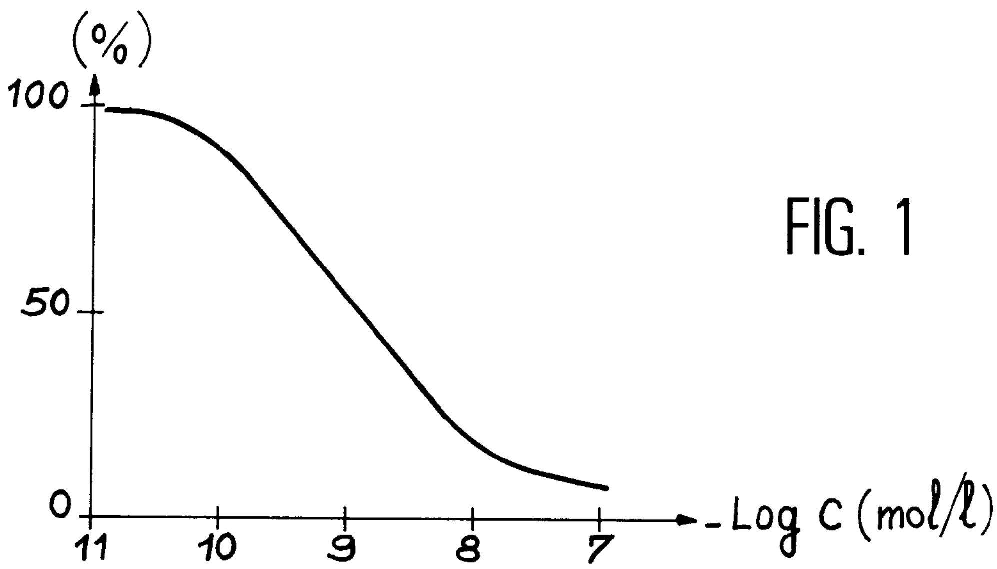


FIG. 1

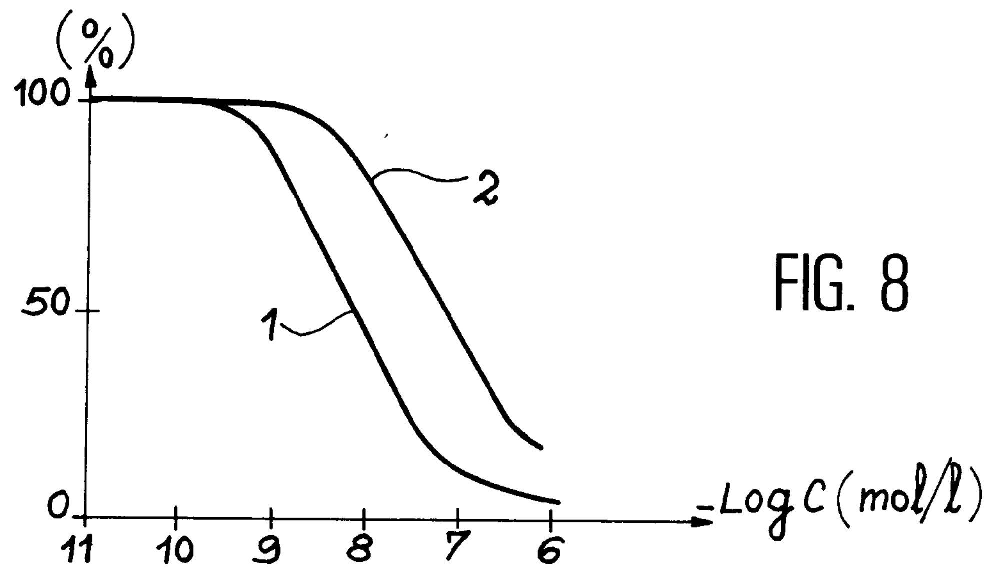


FIG. 8

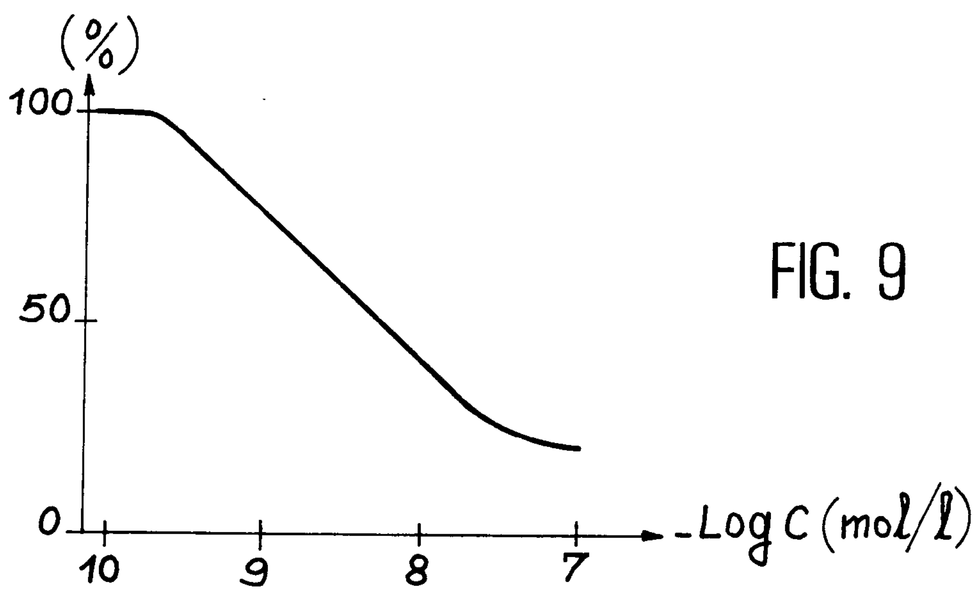


FIG. 9

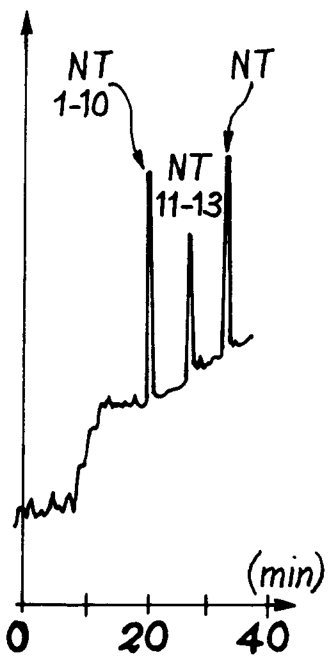


FIG. 2

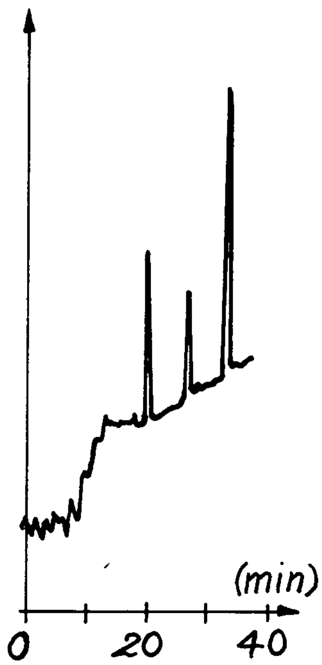


FIG. 3

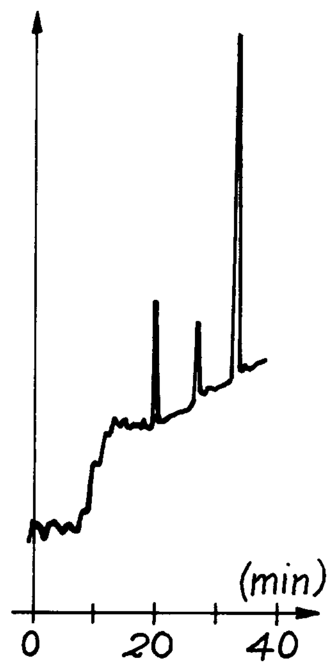


FIG. 4

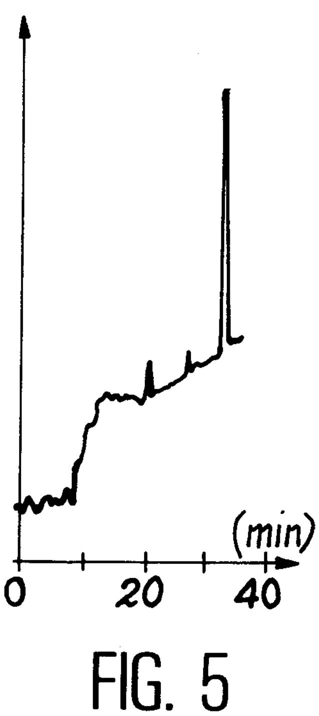


FIG. 5

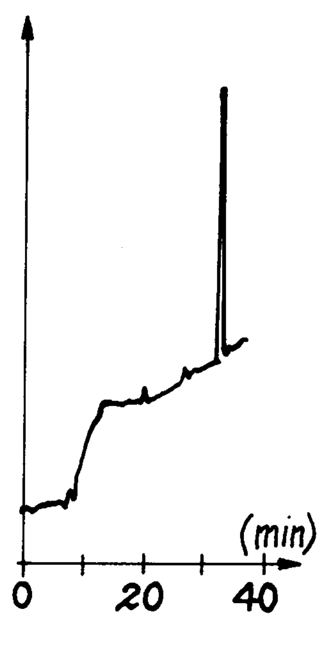


FIG. 6

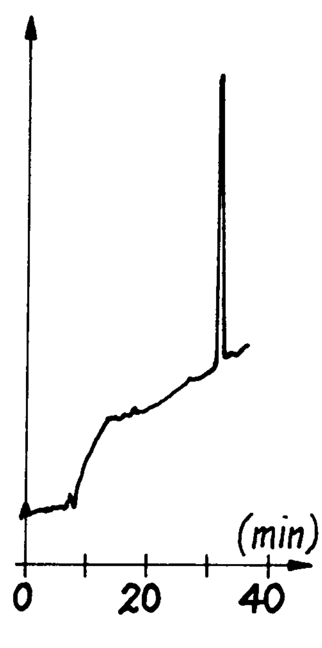


FIG. 7

Office européen des brevets

# RAPPORT DE RECHERCHE EUROPEENNE

Numero de la demande

EP 93 40 0924

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 085 488 (AJINOMOTO CO., INC)<br>* page 1, alinéa 1 - page 4, dernier alinéa *<br>* page 9, dernier alinéa - page 10, alinéa 1 *<br>----- | 1-6 | A61K37/02<br>C07K5/08 |
| | | | **DOMAINES TECHNIQUES RECHERCHES (Int. Cl.5)** |
| | | | C07K<br>A61K |

Le présent rapport a été établi pour toutes les revendications

| Lieu de la recherche | Date d'achèvement de la recherche | Examinateur |
|---|---|---|
| LA HAYE | 03 AOUT 1993 | MONTERO LOPEZ B. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......
& : membre de la même famille, document correspondant

EPO FORM 1503 03.82 (P0402)